# FASCICULE DE BREVET EUROPEEN

(11) **EP 1 564 203 B1**
(45) Date de publication et mention de la délivrance du brevet: **23.12.2009**
(21) Numéro de dépôt: 05290307.7
(22) Date de dépôt: 11.02.2005
(51) Int. Cl.: C07C 253/30, C07C 255/37

(54) **Nouveau procédé de synthèse du (7-méthoxy-1-naphtyl)acétonitrile et application à la synthèse de l'agomélatine**
Neues Verfahren zur Herstellung von (7-Methoxy-1-naphtyl)acetonitril und dessen Anwendung in der Synthese von Agomelatin
New process for synthesizing (7-methoxy-1-naphtyl)acetonitrile and its application in the synthesis of agomelatine

(30) Priorité: 13.02.2004 FR 0401438
(43) Date de publication de la demande: 17.08.2005
(73) Titulaire: Les Laboratoires Servier, 92415 Courbevoie Cédex (FR)
(72) Inventeur: Souvie, Jean-Claude, 76600 Le Havre (FR); Gonzalez Blanco, Isaac, 45002 Toledo (ES)

(56) Documents cités:
- EP-A- 0 447 285
- US-A- 3 931 188
- US-A- 3 992 403
- DEPREUX P ET AL: "SYNTHESIS AND STRUCTURE-ACTIVITY RELATIONSHIPS OF NOVEL NAPHTHALENIC AND BIOISOSTERIC AMIDIC DERIVATIVES AS MELATONIN RECEPTOR LIGANDS" JOURNAL OF MEDICINAL CHEMISTRY, AMERICAN CHEMICAL SOCIETY. WASHINGTON, US, vol. 37, no. 20, 30 septembre 1994 (1994-09-30), pages 3231-3239, XP002016146 ISSN: 0022-2623

## Description

La présente invention concerne un procédé de synthèse industriel du (7-méthoxy-1-naphtyl)acétonitrile, et son application à la production industrielle de l'agomélatine ou N-[2-(7-méthoxy-1-naphtyl)éthyl] acétamide.

Plus spécifiquement, la présente invention concerne un procédé de synthèse industriel du composé de formule (I) :

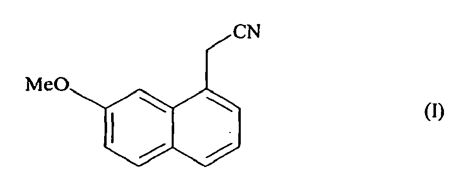

Le composé de formule (I) obtenu selon le procédé de l'invention est utile dans la synthèse de l'agomélatine ou *N*-[2-(7-méthoxy-1-naphtyl)éthyl]acétamide de formule (II) :

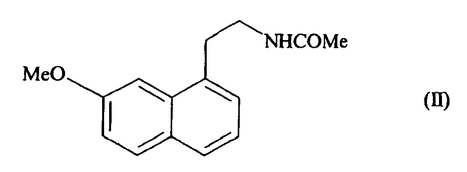

L'agomélatine ou *N*-[2-(7-méthoxy-1-naphtyl)éthyl]acétamide possède des propriétés pharmacologiques intéressantes.

Il présente en effet la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans le brevet européen EP 0 447 285.

Compte-tenu de l'intérêt pharmaceutique de ce composé, il était important de pouvoir y accéder avec un procédé de synthèse industriel performant, facilement transposable à l'échelle industrielle, conduisant à l'agomélatine avec un bon rendement, et une excellente pureté.

Le brevet EP 0 447 285 décrit l'accès en huit étapes à l'agomélatine à partir de la 7-méthoxy-1-tétralone avec un rendement moyen inférieur à 30%.

Ce procédé implique l'action du bromoacétate d'éthyle, suivie d'une aromatisation et saponification pour conduire à l'acide correspondant, qui est ensuite transformé en acétamide puis déshydraté pour conduire au (7-méthoxy-1-naphtyl)acétonitrile, suivie d'une réduction puis de la condensation du chlorure d'acétyle.

En particulier, l'accès au (7-méthoxy-1-naphtyl)acétonitrile implique six étapes réctionnelles et, transposé à l'échelle industrielle, il a rapidement été mis en évidence des difficultés de mise en oeuvre de ce procédé dues principalement à des problèmes de reproductibilité de la première étape constituée par l'action du bromoacétate d'éthyle sur la 7-méthoxy-1-tétralone selon la réaction de Réformatsky conduisant au (7-méthoxy-3,4-dihydro-1 (2*H*)-naphtalenylidène)éthanoate d'éthyle.
De plus, l'étape suivante d'aromatisation du (7-méthoxy-3,4-dihydro-1(2*H*)-naphtalenylidène)éthanoate d'éthyle était souvent partielle et conduisait, après saponification à un mélange de produits difficilement purifiable.

La littérature décrit l'accès en trois étapes au (7-méthoxy-1-naphtyl)acétonitrile à partir de la 7-méthoxy-1-tétralone par action de LiCH₂CN suivie d'une déshydrogénation au DDQ (2,3-dichloro-5,6-dicyano-1,4-benzoquinone) et enfin d'une déshydratation en milieu acide (Synthetic Communication, 2001, 31(4), 621-629). Toutefois le rendement global est moyen (76%) et surtout le DDQ utilisé dans la réaction de déshydrogénation ainsi que le reflux de benzène nécessaire à la troisième étape ne répondent pas aux contraintes industrielles de coût et d'environnement.

La demanderesse a présentement mis au point un nouveau procédé de synthèse industrielle qui conduit, de façon reproductible et sans nécessiter de purification laborieuse, à l'agomélatine avec une pureté qui est compatible avec son utilisation comme principe actif pharmaceutique.

Une alternative aux difficultés rencontrées avec le procédé décrit dans le brevet EP 0 447 285 a été obtenue en condensant directement un dérivé cyano sur la 7-méthoxy-1-tétralone. Il fallait de plus que le composé de condensation obtenu puisse être facilement soumis à une aromatisation afin de conduire au (7-méthoxy-1-naphtyl)acétonitrile sans nécessiter de conditions drastiques et permette l'utilisation de réactifs compatibles avec les exigences industrielles de coût et d'environnement.

Il est apparu que le (7-méthoxy-3,4-dihydro-1-naphtalényl)acétonitrile constituerait un intermédiaire de synthèse idéal répondant aux exigences requises de synthèse directe à partir de la 7-méthoxy-1-tétralone et serait un excellent substrat pour l'étape d'aromatisation.

Des condensations directes de tétralones avec l'acétonitrile ou des dérivés d'acétonitrile sont décrites dans la littérature. En particulier, le brevet US 3,992,403 décrit la condensation de cyanométhylphosphonate sur la 6-fluoro-1-tétralone, et le brevet US 3,931,188 décrit la condensation de l'acétonitrile sur la tétralone conduisant à l'intermédiaire cyané qui est directement engagé dans la réaction suivante.
Appliqué à la 7-méthoxy-1-tétralone, la condensation de l'acétonitrile conduit à un mélange d'isomères « exo » majoritaire et « endo » minoritaire selon la figure 1 : 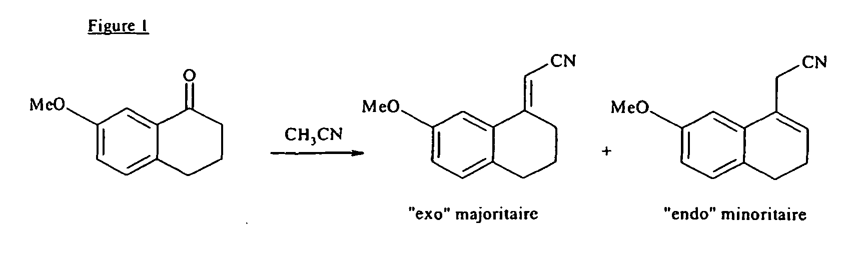 l'obtention d'un tel mélange nécessitant des conditions ultérieures d'aromatisation drastiques non compatibles avec les exigences industrielles pour poursuivre la synthèse de l'agomélatine.

La demanderesse a présentement mis au point un nouveau procédé de synthèse industriel permettant d'obtenir le (7-méthoxy-1-naphtyl)acétonitrile de façon reproductible et sans nécessiter de purification laborieuse, en deux étapes seulement à partir de la 7-méthoxy-tétralone en utilisant comme intermédiaire de synthèse le (7-méthoxy-3,4-dihydro-1-naphtalényl)acétonitrile exempt de l'impureté « exo » de formule (III) : 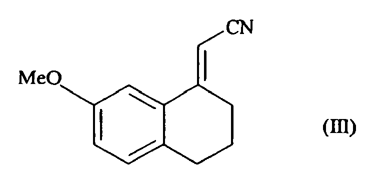 qui ne peut être soumis à la réaction d'aromatisation ultérieure dans des conditions opératoires compatibles avec les exigences industrielles afin de poursuivre la synthèse de l'agomélatine.

Plus spécifiquement, la présente invention concerne un procédé de synthèse industriel du composé de formule (I) : 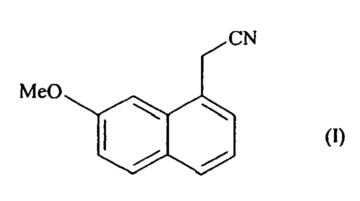 caractérisé en ce que l'on met en réaction la 7-méthoxy-1-tétralone de formule (IV) : 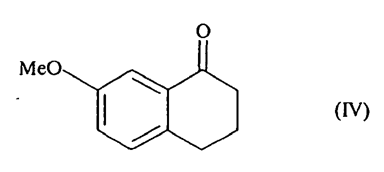 avec l'acide cyanoacétique de formule (V) : 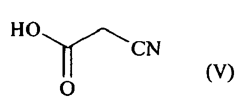 dans des conditions d'élimination de l'eau formée, en présence d'une quantité catalytique du composé de formule (VI) : 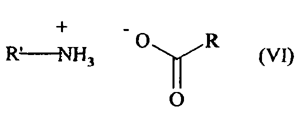 dans laquelle R et R', identiques ou différents, représentent chacun un groupement alkyle (C₃-C₁₀) linéaire ou ramifié, un groupement aryle non substitué ou substitué, ou un groupement arylalkyle (C₁-C₆) linéaire ou ramifié non substitué ou substitué,
pour conduire après filtration et lavage par une solution basique au (7-méthoxy-3,4-dihydro-1-naphtalényl)acétonitrile de formule (VII) : 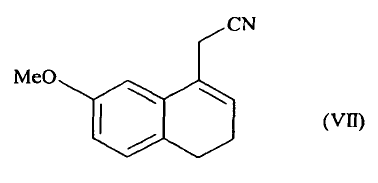 composé de formule (VII) qui est mis en réaction avec un catalyseur d'hydrogénation en présence d'un dérivé allylique,
pour conduire au composé de formule (I) après filtration et évaporation du solvant, composé de formule (I) que l'on isole sous la forme d'un solide après recristallisation,
étant entendu que :
- par aryle on entend un groupement phényle, naphtyle ou biphényle,
- le terme « substitué » affecté aux expressions « aryle » et « arylalkyle » signifie que la partie aromatique de ces groupements peut être substituée par 1 à 3 groupements, identiques ou différents, choisis parmi alkyle (C₁-C₆) linéaire ou ramifié, hydroxy, et alkoxy (C₁-C₆) linéaire ou ramifié,
- par « dérivé allylique » on entend toute molécule contenant 3 à 10 atomes de carbone et pouvant contenir en plus 1 à 5 atomes d'oxygène, et contenant au moins un motif-CH₂-CH=CH₂.

Plus particulièrement, dans la réaction de transformation du composé de formule (IV) en composé de formule (VII), l'eau formée est éliminée par distillation. On utilise préférentiellement un solvant de réaction ayant une température d'ébullition supérieure ou égale à celle de l'eau et encore plus préférentiellement formant un azéotrope avec l'eau comme par exemple le xylène, le toluène, l'anisole, l'éthylbenzène, le tétrachloroéthylène, le cyclohexène, ou le mésitylène.
De façon préférée, la réaction de transformation du composé de formule (IV) en composé de formule (VII) est réalisée au reflux du toluène ou du xylène et plus préférentiellement au reflux du toluène.

Avantageusement, dans la réaction de transformation du composé de formule (IV) en composé de formule (VII), l'un des groupements R ou R' du catalyseur utilisé représente un groupement alkyle (C₃-C₁₀) linéaire ou ramifié, et l'autre représente un groupement aryle ou arylalkyle. Plus particulièrement, un catalyseur préféré est celui de formule (VIₐ) : 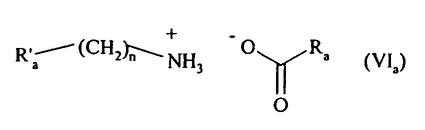 dans laquelle R'ₐ représente un groupement phényle non substitué ou substitué par un ou plusieurs groupements alkyle (C₁-C₆) linéaire ou ramifié, n vaut 0 ou 1, et Rₐ représente un groupement alkyle (C₃-C₁₀) linéaire.
Très avantageusement, Rₐ' représente un groupement phényle non substitué ou substitué et plus particulièrement un groupement phényle non substitué.

Le groupement Rₐ préféré est le groupement hexyle.

Avantageusement, n vaut 1.

Le catalyseur préféré utilisé dans la réaction de transformation du composé de formule (IV) en composé de formule (VII) selon le procédé de l'invention est l'heptanoate de benzylammonium de formule (VIII) :

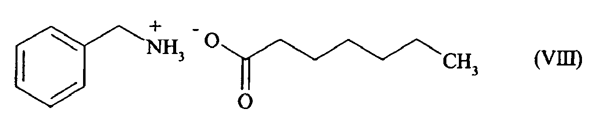

Avantageusement, le composé de formule (VII) est obtenu après filtration et lavage par une solution basique organique ou minérale comme NaOH, KOH, Ca(OH)₂, Sr(OH)₂, ou NH₄OH, et plus préférentiellement par une solution d'hydroxyde de sodium.

De façon préférentielle, la réaction de transformation du composé de formule (VII) en composé de formule (I) est réalisée au reflux du toluène ou du xylène et plus préférentiellement au reflux du toluène.

Le catalyseur utilisé préférentiellement dans la réaction de transformation du composé de formule (VII) en composé de formule (I) est un catalyseur sous forme d'oxyde ou supporté comme par exemple le palladium, le platine, le nickel, Al₂O₃ et plus particulièrement le palladium. Avantageusement, on utilise le palladium sur charbon, plus particulièrement le palladium sur charbon de 1 à 20% et encore plus particulièrement à 5% ou 10%. Préférentiellement, on utilisera du palladium sur charbon dans des quantités catalytiques, plus particulièrement dans des quantités allant de 1 à 10% en poids de catalyseur par rapport au poids de substrat et plus préférentiellement 5%.

L'accepteur d'hydrogène utilisé préférentiellement dans la réaction de transformation du composé de formule (VII) en composé de formule (I) est un dérivé allylique et plus particulièrement un acrylate d'allyle ou un allylglycidyléther. L'acrylate d'allyle préféré du procédé selon l'invention est le méthacrylate d'allyle.

Ce procédé est particulièrement intéressant pour les raisons suivantes :
- il permet d'obtenir à l'échelle industrielle le composé « endo » de formule (VII) de façon exclusive. Ce résultat est tout à fait surprenant lorsqu'on considère la littérature concernant ce type de réaction qui fait le plus souvent état de l'obtention de mélanges « exo »/ « endo » (Tetrahedron, 1966, 22, 3021-3026). Ce résultat provient de l'utilisation dans la réaction d'un catalyseur de formule (VI) en lieu et place des acétates d'ammonium couramment utilisés dans ces réactions (Bull. Soc. Chim. Fr., 1949, 884-890).
- le taux de transformation du composé de formule (IV) en composé de formule (VII) obtenu est très élevé, supérieur à 97% contrairement à ce qui a pu être observé avec l'utilisation d'acide acétique pour lequel ce taux ne dépasse pas 75%.
- l'utilisation d'un catalyseur d'hydrogénation en présence d'un dérivé allylique et plus particulièrement du méthacrylate d'allyle pour la transformation du composé de formule (VII) en composé de formule (I) est tout à fait compatible avec les exigences industrielles de coût et d'environnement, contrairement aux quinones couramment utilisées.
- il permet de plus d'obtenir à l'échelle industrielle le composé de formule (I) de façon exclusive, en particulier exempt du produit de réduction correspondant de formule (IX) : 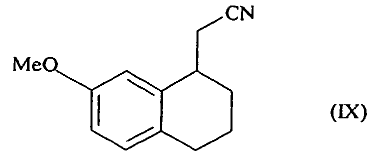
- enfin les taux de transformations du composé de formule (VII) en composé de formule (I) observés sont élevés, supérieurs à 90%.

Le composé de formule (VII) obtenu selon le procédé de l'invention est nouveau et est utile en tant qu'intermédiaire de synthèse de l'agomélatine dans laquelle il est soumis à une réaction d'aromatisation suivie d'une réaction de réduction puis de couplage avec l'anhydride acétique.

Le composé de formule (I) ainsi obtenu est, le cas échéant, soumis à une réduction puis à une réaction de couplage avec l'anhydride acétique pour conduire à l'agomélatine.

Les exemples ci-dessous illustrent l'invention.

### Exemple 1 : (7-Méthoxy-1-naphtyl)acétonitrile

### Stade A : (7-Méthoxy-3,4-dihydro-1-naphtalényl)acétonitrile

Dans un réacteur de 670 l sont introduits 85,0 kg de 7-méthoxy-1-tétralone, 60,3 kg d'acide cyanoacétique et 15,6 kg d'acide heptanoïque dans du toluène en présence de 12,7 kg de benzylamine. Le milieu est porté à reflux. Lorsque tout le substrat de départ a disparu, la solution est refroidie et filtrée. Le précipité obtenu est lavé par du toluène puis le filtrat obtenu est lavé par une solution de soude 2N, puis par de l'eau jusqu'à neutralité. Après évaporation du solvant, le solide obtenu est recristallisé dans un mélange éthanol/eau (80/20) pour conduire au produit du titre avec un rendement de 90 % et une pureté chimique supérieure à 99%.

### Point de fusion : 48-50°C

### Stade B : (7-Méthoxy-1-naphtyl)acétonitrile

Dans un réacteur de 670 l sont introduits 12,6 kg de palladium sur charbon à 5% dans du toluène et portés à reflux, puis 96,1 kg de (7-méthoxy-3,4-dihydro-1-naphtalényl) acétonitrile en solution dans du toluène sont ajoutés ainsi que 63,7 kg de méthacrylate d'allyle. La réaction se poursuit à reflux et est suivie par chromatographie en phase vapeur. Lorsque tout le substrat de départ a disparu, le milieu réactionnel est refroidi à l'ambiante puis filtré. Après évaporation du toluène, le résidu solide obtenu est recristallisé dans un mélange éthanol/eau (80/20) pour conduire au produit du titre avec un rendement de 91 % et une pureté chimique supérieure à 99%.

### Point de fusion : 83°C

### Exemple 2 : (7-Méthoxy-1-naphtyl)acétonitrile

### Stade A : (7-Méthoxy-3,4-dihydro-1-naphtalényl)acétonitrile

Dans un réacteur de 670 l sont introduits 85,0 kg de 7-méthoxy-1-tétralone, 60,3 kg d'acide cyanoacétique et 15,6 kg d'acide heptanoïque dans du toluène en présence de 11,0 kg d'aniline. Le milieu est porté à reflux. Lorsque tout le substrat de départ a disparu, la solution est refroidie et filtrée. Le précipité obtenu est lavé par du toluène puis le filtrat obtenu est lavé par une solution de soude 2N, puis par de l'eau jusqu'à neutralité. Après évaporation du solvant, le solide obtenu est recristallisé dans un mélange éthanol/eau (80/20) pour conduire au produit du titre avec un rendement de 87% et une pureté chimique supérieure à 99%.

### Point de fusion : 48-50°C

### Stade B : (7 Méthoxy-1-naphtyl)acétonitrile

On procède comme dans le stade B de l'Exemple 1.

## Revendications

1. Procédé de synthèse industriel du composé de formule (I) 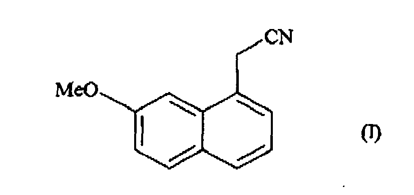 **caractérisé en ce que** l'on met en réaction la 7-méthoxy-1-tétralone de formule (IV) : 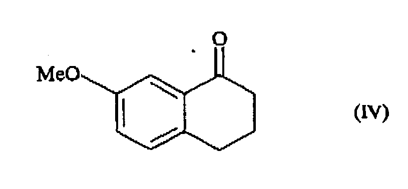 avec l'acide cyanoacétique de formule (V) : 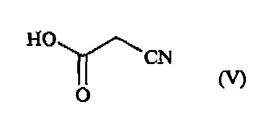 dans des conditions d'élimination de l'eau formée, en présence d'une quantité catalytique du composé de formule (VI) : 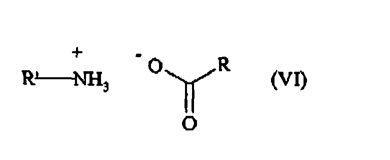 dans laquelle R et R', identiques ou différents, représentent chacun un groupement alkyle (C₃-C₁₀) linéaire ou ramifié, un groupement aryle non substitué ou substitué, ou un groupement arylalkyle (C₁-C₆) linéaire ou ramifié non substitué ou substitué,
pour conduire après filtration et lavage par une solution basique au (7-méthoxy-3,4-dihydro-1-naphtalényl)acétomtrile de formule (VII) : 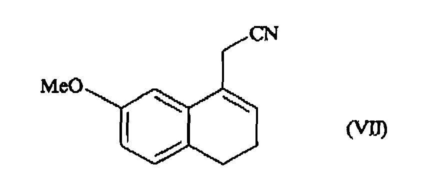 composé de formule (VII) qui est mis en réaction avec un catalyseur d'hydrogénation en présence d'un dérivé allylique,
pour conduire au composé de formule (I) après filtration et évaporation du solvant, composé de formule (I) que l'on isole sous la forme d'un solide après recristallisation,
étant entendu que :
- par aryle on entend un groupement phényle, naphtyle ou biphényle,
- le terme « substitué » affecté aux expressions « aryle » et « arylalkyle » signifie que la partie aromatique de ces groupements peut être substituée par 1 à 3 groupements, identiques ou différents, choisis parmi alkyle (C₁-C₆) linéaire ou ramifié, hydroxy, et alkoxy (C₁-C₆) linéaire ou ramifié
- par « dérivé allylique » on entend toute molécule contenant 3 à 10 atomes de carbone et pouvant contenir en plus 1 à 5 atomes d'oxygène, et contenant au moins un motif -CH₂-CH=CH₂.

2. Procédé de synthèse du composé de formule (I) selon la revendication 1 **caractérisé en ce que** la réaction de transformation du composé de formule (IV) en composé de formule (VII) est réalisée au reflux du toluène.

3. Procédé de synthèse du composé de formule (I) selon la revendication 1, **caractérisé en ce que** le catalyseur utilisé pour la réaction de transformation du composé de formule (IV) en composé de formule (VII) est le composé de formule (VIₐ) : 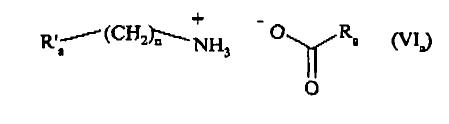 dans laquelle R'ₐ représente un groupement phényle non substitué ou substitué par un ou plusieurs groupements alkyle (C₁-C₆) linéaire ou ramifié, n vaut 0 ou 1, et Rₐ représente un groupement alkyle (C₃-C₁₀) linéaire.

4. Procédé de synthèse du composé de formule (I) selon la revendication 1, **caractérisé en ce que** R représente un groupement hexyle.

5. Procédé de synthèse du composé de formule (I) selon la revendication 1, **caractérisé en ce que** R' représente un groupement benzyle.

6. Procédé de synthèse du composé de formule (I) selon la revendication 1, **caractérisé en ce que** le catalyseur utilisé pour la réaction de transformation du composé de formule (IV) en composé de formule (VII) est l'heptanoate de benzylammonium de formule (VII) :

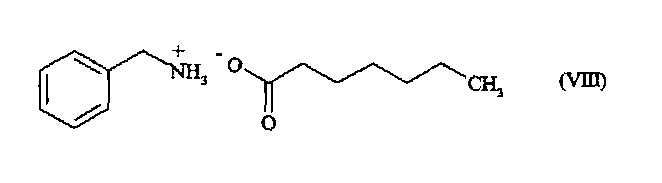

7. Procédé de synthèse du composé de formule (I) selon la revendication 1 **caractérisé en ce que** la réaction de transformation du composé de formule (VII) en composé de formule (I) est réalisée au reflux du toluène.

8. Procédé de synthèse du composé de formule (I) selon la revendication 1, **caractérisé en ce que** le catalyseur d'hydrogénation utilisé dans la réaction de transformation du composé de formule (VII) en composé de formule (I) est le palladium.

9. Procédé de synthèse du composé de formule (I) selon la revendication 1, **caractérisé en ce que** le catalyseur d'hydrogénation utilisé dans la réaction de transformation du composé de formule (VII) en composé de formule (I) est le palladium sur charbon à 5%.

10. Procédé de synthèse du composé de formule (I) selon la revendication 1, **caractérisé en ce que** la quantité de catalyseur d'hydrogénation utilisée dans la réaction de transformation du composé de formule (VII) en composé de formule (I) est de 5% en poids de catalyseur par rapport au poids de substrat.

11. Procédé de synthèse de l'agomélatine à partir du composé de formule (I), **caractérisé en ce que** le composé de formule (I) est obtenu par le procédé de synthèse selon l'une quelconque des revendications 1 à 6 et 7 à 10, et que l'on soumet à une réduction puis à un couplage avec l'anhydride acétique.

## Claims

1. Process for the industrial synthesis of the compound of formula (I) 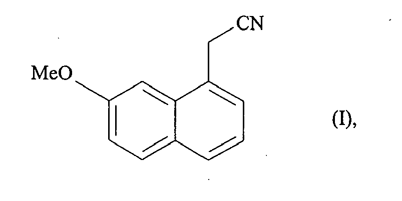 **characterised in that** 7-methoxy-1-tetralone of formula (IV) : 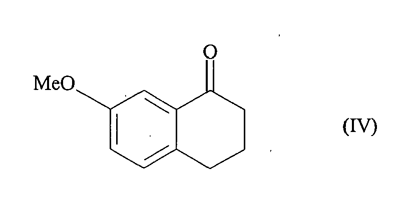 is reacted with cyanoacetic acid of formula (V) : 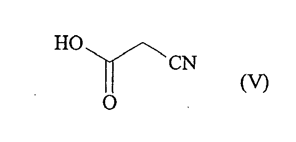 under conditions of removal of the water formed, in the presence of a catalytic amount of a compound of formula (VI) : 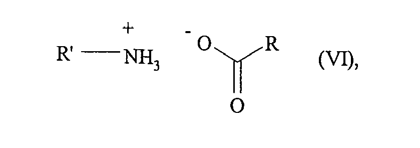 wherein R and R', which may be the same or different, each represent a linear or branched (C₃-C₁₀)alkyl group, an unsubstituted or substituted aryl group or an unsubstituted or substituted aryl-(C₁-C₆)alkyl group in which the alkyl moiety is linear or branched,
to yield, after filtration and washing with a basic solution, (7-methoxy-3,4-dihydro-1-naphthalenyl)acetonitrile of formula (VII) : 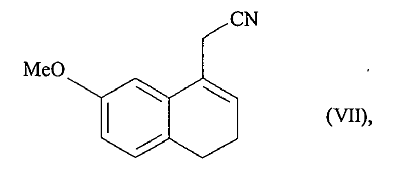 which compound of formula (VII) is reacted with a hydrogenation catalyst in the presence of an allylic compound
to yield the compound of formula (I) after filtration and removal of the solvent by evaporation, which compound of formula (I) is isolated in the form of a solid after recrystallisation,
it being understood that:
- aryl means a phenyl, naphthyl or biphenyl group,
- the term "substituted" relating to the expressions "aryl" and "arylalkyl" means that the aromatic moiety of these groups may be substituted by 1 to 3 identical or different groups selected from linear or branched (C₁-C₆)alkyl, hydroxy and linear or branched (C₁-C₆)alkoxy,
- an "allylic compound" means any molecule which contains 3 to 10 carbon atoms and which may additionally contain 1 to 5 oxygen atoms, and which contains at least one -CH₂-CH=CH₂ moiety.

2. Process for the synthesis of the compound of formula (I) according to claim 1, **characterised in that** the reaction converting the compound of formula (IV) to the compound of formula (VII) is carried out with reflux of toluene.

3. Process for the synthesis of the compound of formula (I) according to claim 1, **characterised in that** the catalyst used for the reaction converting the compound of formula (IV) to the compound of formula (VII) is the compound of formula (VIₐ): 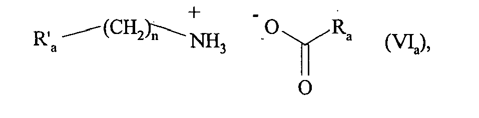 wherein R'ₐ represents a phenyl group unsubstituted or substituted by one or more linear or branched (C₁-C₆)alkyl groups, n is 0 or 1, and Rₐ represents a linear (C₃-C₁₀)alkyl group.

4. Process for the synthesis of the compound of formula (I) according to claim 1, **characterised in that** R represents a hexyl group.

5. Process for the synthesis of the compound of formula (I) according to claim 1, **characterised in that** R' represents a benzyl group.

6. Process for the synthesis of the compound of formula (I) according to claim 1, **characterised in that** the catalyst used for the reaction converting the compound of formula (IV) to the compound of formula (VII) is benzylammonium heptanoate of formula (VIII) :

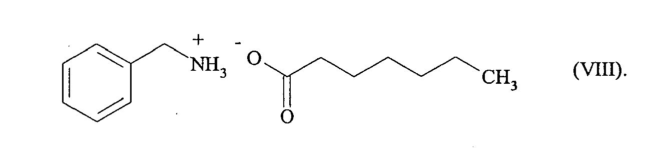

7. Process for the synthesis of the compound of formula (I) according to claim 1, **characterised in that** the reaction converting the compound of formula (VII) to the compound of formula (I) is carried out with reflux of toluene.

8. Process for the synthesis of the compound of formula (I) according to claim 1, **characterised in that** the hydrogenation catalyst used in the reaction converting the compound of formula (VII) to the compound of formula (I) is palladium.

9. Process for the synthesis of the compound of formula (I) according to claim 1, **characterised in that** the hydrogenation catalyst used in the reaction converting the compound of formula (VII) to the compound of formula (I) is 5% palladium-on-carbon.

10. Process for the synthesis of the compound of formula (I) according to claim 1, **characterised in that** the amount of hydrogenation catalyst used in the reaction converting the compound of formula (VII) to the compound of formula (I) is 5% by weight of catalyst in relation to the weight of substrate.

11. Process for the synthesis of agomelatine starting from the compound of formula (I), **characterised in that** the compound of formula (I) is obtained by the synthesis process according to any one of claims 1 to 6 and 7 to 10, and is subjected to reduction and then to coupling with acetic anhydride.

## Patentansprüche

1. Verfahren zur industriellen Synthese der Verbindung der Formel (I) 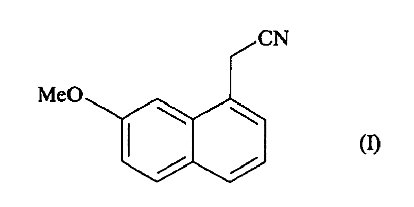 ***dadurch gekennzeichnet*, dass** man 7-Methoxy-1-tetralon der Formel (IV): 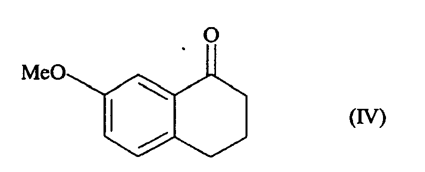 mit Cyanessigsäure der Formel (V): 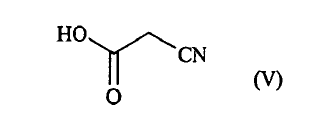 unter den Bedingungen der Eliminierung des gebildeten Wassers in Gegenwart einer katalytischen Menge der Verbindung der Formel(VI): 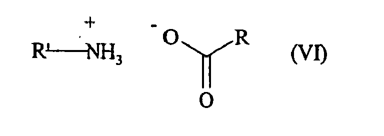 in der R und R', die identisch oder verschieden sind, jeweils eine geradkettige oder verzweigte (C₃-C₁₀)-Alkylgruppe, eine nichtsubstituierte oder substituierte Arylgruppe oder eine geradkettige oder verzweigte, nichtsubstituierte oder substituierte Aryl-(C₁-C₆)-alkylgruppe bedeuten, umsetzt,
sodass man nach der Filtration und dem Waschen mit einer basischen Lösung (7-Methoxy-3,4-dihydro-1-naphthalinyl)-acetonitril der Formel (VII) erhält: 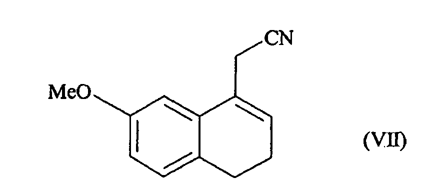 welche Verbindung der Formel (VII) in Gegenwart eines Allylderivats mit einem Hydrierkatalysator umgesetzt wird,
sodass man nach der Filtration und dem Verdampfen des Lösungsmittels die Verbindung der Formel (I) erhält, welche Verbindung der Formel (I) man nach der Umkristallisation in Form eines Feststoffs isoliert,
mit der Maßgabe, dass:
- man unter Aryl eine Phenyl-, Naphthyl- oder Biphenylgruppe versteht,
- der Begriff «substituiert» im Hinblick auf die Begriffe «Aryl» und «Arylalkyl» bedeutet, dass der aromatische Teil dieser Gruppen durch 1 bis 3 identische oder verschiedenartige Gruppen ausgewählt aus geradkettigem oder verzweigtem (C₁-C₆)-Alkyl, Hydroxy und geradkettigem oder verzweigtem (C₁-C₆)-Alkoxy substituiert sein kann und
- man unter «Allylderivat» jedes Molekül versteht, das 3 bis 10 Kohlenstoffatome enthält und zusätzlich 1 bis 5 Sauerstoffatome enthalten kann und mindestens einen Rest der Formel -CH₂-CH=CH₂ aufweist.

2. Verfahren zur Synthese der Verbindung der Formel (I) nach Anspruch 1, **dadurch gekennzeichnet, dass** die Reaktion der Umwandlung der Verbindung der Formel (IV) in die Verbindung der Formel (VII) in Toluol am Rückfluss durchgeführt wird.

3. Verfahren zur Synthese der Verbindung der Formel (I) nach Anspruch 1, **dadurch gekennzeichnet, dass** der für die Reaktion der Umwandlung der Verbindung der Formel (IV) in die Verbindung der Formel (VII) verwendete Katalysator die Verbindung der Formel (VIₐ) ist: 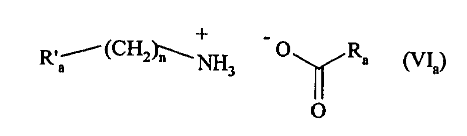 in der R'ₐ eine Phenylgruppe bedeutet, die nicht substituiert ist oder durch eine oder mehrere geradkettige oder verzweigte (C₁-C₆)-Alkylgruppen substituiert ist, n den Wert 0 oder 1 besitzt und Rₐ eine geradkettige (C₃-C₁₀)-Alkylgruppe bedeutet.

4. Verfahren zur Synthese der Verbindung der Formel (I) nach Anspruch 1, **dadurch gekennzeichnet, dass** R eine Hexylgruppe bedeutet.

5. Verfahren zur Synthese der Verbindung der Formel (I) nach Anspruch 1, **dadurch gekennzeichnet, dass** R' eine Benzylgruppe bedeutet.

6. Verfahren zur Synthese der Verbindung der Formel (I) nach Anspruch 1, **dadurch gekennzeichnet, dass** der für die Reaktion der Umwandlung der Verbindung der Formel (IV) in die Verbindung der Formel (VII) verwendete Katalysator Benzylammoniumheptanoat der Formel (VIII) ist:

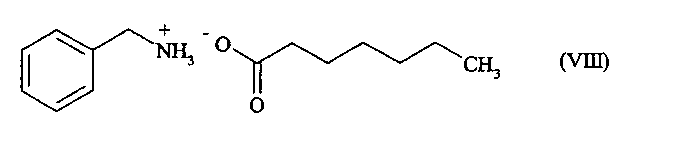

7. Verfahren zur Synthese der Verbindung der Formel (I) nach Anspruch 1, **dadurch gekennzeichnet, dass** die Reaktion der Umwandlung der Verbindung der Formel (VII) in die Verbindung der Formel (I) in Toluol am Rückfluss durchgeführt wird.

8. Verfahren zur Synthese der Verbindung der Formel (I) nach Anspruch 1, **dadurch gekennzeichnet, dass** der bei der Reaktion der Umwandlung der Verbindung der Formel (VII) in die Verbindung der Formel (I) verwendete Hydrierkatalysator Palladium ist.

9. Verfahren zur Synthese der Verbindung der Formel (I) nach Anspruch 1, **dadurch gekennzeichnet, dass** der für die Reaktion der Umwandlung der Verbindung der Formel (VII) in die Verbindung der Formel (I) verwendete Hydrierkatalysator 5 % Palladium-auf-Kohlenstoff ist.

10. Verfahren zur Synthese der Verbindung der Formel (I) nach Anspruch 1, **dadurch gekennzeichnet, dass** die Menge des bei der Reaktion der Umwandlung der Verbindung der Formel (VII) in die Verbindung der Formel (I) verwendeten Hydrierkatalysators 5 Gew.-% Katalysator, bezogen auf das Gewicht des Substrats, beträgt.

11. Verfahren zur Synthese von Agomelatin ausgehend von der Verbindung der Formel (I), **dadurch gekennzeichnet, dass** die Verbindung der Formel (I) nach dem Syntheseverfahren nach einem der Ansprüche 1 bis 6 und 7 bis 10 hergestellt wird und dann einer Reduktion und schließlich einer Kupplung mit Essigsäureanhydrid unterworfen wird.
